# EUROPEAN PATENT SPECIFICATION

(11) **EP 4 122 335 B1**
(45) Date of publication and mention of the grant of the patent: **15.07.2026**
(21) Application number: 20926191.6
(22) Date of filing: 25.12.2020
(51) Int. Cl.: A24F 40/40, A61M 11/04, A24F 40/46, H05B 3/42, H05B 3/20, H05B 3/04, A61M 15/06, A24F 40/85, A24F 40/20, A61M 15/00

(54) **ELECTRIC HEATING NON-COMBUSTION ATOMIZATION DEVICE**
VERBRENNUNGSFREIE ZERSTÄUBUNGSVORRICHTUNG MIT ELEKTRISCHER HEIZUNG
DISPOSITIF ÉLECTRIQUE D'ATOMISATION PAR CHAUFFAGE SANS COMBUSTION

(30) Priority: 20.03.2020 CN 202020373424 U
(43) Date of publication of application: 25.01.2023
(73) Proprietor: Shenzhen Merit Technology Co., Ltd., Shenzhen, Guangdong 518105 (CN)
(72) Inventor: ZHANG, Xingfu, Shenzhen, Guangdong 518105 (CN); YU, Xiaochuan, Shenzhen, Guangdong 518105 (CN)
(74) Representative: Herrero & Asociados, S.L.
(86) International application number: PCT/CN2020/139457
(87) International publication number: WO 2021/184876

(56) References cited:
- CN-A- 106 072 772
- CN-A- 109 007 986
- CN-A- 109 938 411
- CN-A- 110 786 549
- CN-U- 203 103 593
- CN-U- 205 390 306
- CN-U- 208 129 432
- CN-U- 208 490 851
- CN-U- 209 121 288
- CN-U- 209 314 965
- CN-U- 209 825 215
- CN-U- 210 017 863
- CN-U- 210 076 577
- CN-U- 210 124 318
- CN-U- 210 130 349
- KR-B1- 101 772 344
- US-A1- 2019 208 825

## Description

### TECHNICAL FIELD

The present disclosure relates to the field of electronic atomization technologies, and in particular to an electronic heating non-combustion atomization device.

### BACKGROUND

Currently, there are some not-burn and low-temperature electronic atomization devices in the market, the principle of which is to heat the solid aerosol-forming substrate at the low temperature, thereby generating smoke to be inhaled by a user.

However, an electronic atomization device of the related art usually includes a plurality of components having complicated connection relationships. When some of the components fail, the user cannot maintain or replace them and have to throw away the entire electronic atomization device, resulting in high use costs.

CN209314965U relates to an electronic cigarette with a replaceable battery, which includes an atomizer, a host and a power supply structure. A battery is arranged in the power supply structure, the atomizer is connected to the host through a conductive element, the host is connected to the power supply structure through a conductive element, and the atomizer and the power supply structure are respectively plugged at two ends of a second shell.

### SUMMARY

The present invention provides an electronic heating non-combustion atomization device as set out in the appended set of claims.

In the embodiments of the present disclosure, the circuit board assembly, the heating atomization assembly, and the battery assembly are set as independent models. The battery assembly is fixed to the housing, the heating atomization assembly is at least partially received in the first end of the housing, the battery assembly is at least partially received in the second end of the housing, the heating atomization assembly and the battery assembly are detachably connected to the circuit board assembly so as to be able to be taken out of the housing. Therefore, it is convenient to assemble and disassemble each model, and it is convenient to maintain the electronic heating non-combustion atomization device and replace the component of the electronic heating non-combustion atomization device, thereby prolonging the service life of the electronic heating non-combustion atomization device.

### BRIEF DESCRIPTION OF THE DRAWINGS

FIG. 1 is a structural schematic view of an electronic heating non-combustion atomization device according to an embodiment of the present disclosure.
FIG. 2 is an exploded structural schematic view of the electronic heating non-combustion atomization device according to an embodiment of the present disclosure.
FIG. 3 is a sectional structural schematic view of the electronic heating non-combustion atomization device according to an embodiment of the present disclosure.
FIG. 4 is an exploded structural schematic view of a circuit board assembly of the electronic heating non-combustion atomization device according to an embodiment of the present disclosure.
FIG. 5 is an exploded structural schematic view of a heating atomization assembly of the electronic heating non-combustion atomization device.

### DETAILED DESCRIPTION

The technical solutions in the embodiments of the present disclosure will be clearly and completely described below in conjunction with the accompanying drawings in the embodiments of the present disclosure. It is clear that the embodiments described are only a part of the embodiments of the present disclosure, and not all of them.

The terms "first", "second", and so on in the present disclosure are intended for descriptive purposes only and are not to be construed as indicating or implying relative importance or implicitly specifying the number of indicated technical features. Thus, a feature qualified with "first" or "second" may either explicitly or implicitly indicate that at least one such feature is included. In the description of the present disclosure, "a plurality" means at least two, e.g., two, three, etc., unless otherwise expressly and specifically limited. All directional indications (e.g., up, down, left, right, forward, backward, ...) in the present disclosure are intended only to explain the relative position relationship, movement, etc., between assemblies in a particular posture (as shown in the accompanying drawings). When the particular posture is changed, the directional indications are changed accordingly. In addition, the terms "include" and "have" and any variations thereof are intended to cover non-exclusive inclusion. For example, a process, method, system, product, or apparatus including a series of steps or units is not limited to the listed steps or units, but optionally also includes steps or units not listed, or optionally also includes other steps or units inherent to the process, method, product, or apparatus.

References herein to "embodiments" mean that particular features, structures, or characteristics described in connection with an embodiment may be included in at least one embodiment of the present disclosure. The presence of the phrase at various points in the specification does not necessarily mean a same embodiment, nor is it a separate or alternative embodiment that is mutually exclusive with other embodiments. It is understood, both explicitly and implicitly, by those skilled in the art that the embodiments described herein may be combined with other embodiments.

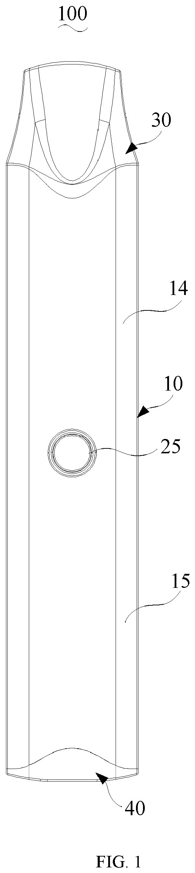

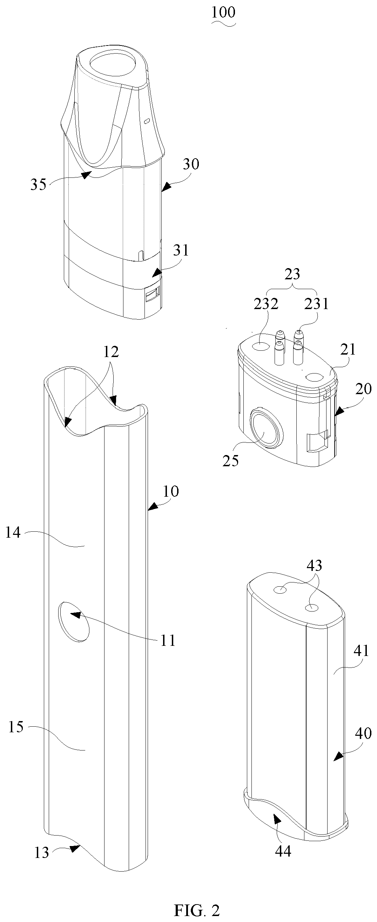

Referring to FIG. 1 and FIG. 2, FIG. 1 is a structural schematic view of an electronic heating non-combustion atomization device according to an embodiment of the present disclosure. FIG. 2 is an exploded structural schematic view of the electronic heating non-combustion atomization device according to an embodiment of the present disclosure.

The electronic heating non-combustion atomization device 100 includes a hollow housing 10, a circuit board assembly 20, a heating atomization assembly 30, and a battery assembly 40.

The circuit board assembly 20 is fixedly disposed in the housing 10.

The circuit board assembly 20 may be fixedly connected to the inner sidewall of the housing 10, and the circuit board assembly 20 may be fixed to a middle of the housing 10, such that two ends of the housing 10 may accommodate other assemblies. The two ends of the housing 10 include a first end 14 disposed in one side of the circuit board assembly 20 and a second end 15 disposed in the other side of the circuit board assembly 20. The hollow housing 10 may a part of the outer housing of the electronic heating non-combustion atomization device 100.

The heating atomization assembly 30 is configured to heat an aerosol-forming substrate. The heating atomization assembly 30 is at least partially received in the first end 14 of the housing 10. The heating atomization assembly 30 is detachably connected to the circuit board assembly 20, such that the heating atomization assembly 30 may be removed from the housing 10.

The aerosol-forming substrate may be a cigarette, a cartridge, a drug, or the like.

In the embodiment, the heating atomization assembly 30 may be detachably connected to the circuit board assembly 20, and detachable from the housing 10, such that the heating atomization assembly 30 may be relatively fixed and separated from the circuit board assembly 20. Thus, it is convenient to assemble and disassemble, and the heating atomization assembly 30 may be taken out of the housing 10. The heating atomization assembly 30 is electrically connected to the circuit board assembly 20 through a conductive terminal, contactor, or the like. When the heating atomization assembly 30 is connected to the circuit board assembly 20, the electrical connection is correspondingly achieved. When the heating atomization assembly 30 is detachable from the circuit board assembly 20, the electrical connection is correspondingly broken down.

The battery assembly 40 is configured to provide power. The battery assembly 40 is at least partially received in the second end 15 of the housing 10. The battery assembly 40 is detachably connected to the circuit board assembly 20, such that the battery assembly 40 may be removed from the housing 10.

The battery assembly 40 is configured to supply power for the circuit board assembly 20 and the heating atomization assembly 30 of the electronic heating non-combustion atomization device 100.

In the embodiment, the battery assembly 40 may be detachably connected to the circuit board assembly 20, and detachable from the housing 10, such that the battery assembly 40 may be relatively fixed and separated from the circuit board assembly 20. Thus, it is convenient to assemble and disassemble, and the battery assembly 40 may be taken out of the housing 10. Likewise, the battery assembly 40 is electrically connected to the circuit board assembly 20 through the conductive terminal, the contactor, or the like. When the battery assembly 40 is connected to the circuit board assembly 20, the electrical connection is correspondingly achieved. When the battery assembly 40 is detachable from the circuit board assembly 20, the electrical connection is correspondingly broken down.

Optionally, the heating atomization assembly 30 and the battery assembly 40 are electrically connected to the circuit board assembly 20 via an electrode. The electrode may be the conductive terminal, the contactor, or the like.

In the embodiment, the hollow housing 10 is configured to fix the circuit board assembly 20. Furthermore, it is convenient for the user to partially accommodate the heating atomization assembly 30 and the battery assembly 40 in the housing 10 by means of insertion, such that the heating atomization assembly 30 and the battery assembly 40 are electrically connected to the circuit board assembly 20, thus improving the entire structural stability of the electronic heating non-combustion atomization device 100 through the housing 10.

When in use, the circuit board assembly 20 has been arranged in the housing 10, the heating atomization assembly 30 is partially inserted into the first end 14 of the housing 10 and is detachably connected to the circuit board assembly 20, and then the battery assembly 40 is partially inserted into the second end 15 of the housing 10 and is detachably connected to the circuit board assembly 20 so as to complete the assembly. The assembled heating atomization assembly 30 and battery assembly 40 may be electrically connected with the circuit board assembly 20. After the electronic heating non-combustion atomization device 100 is used for a period of time, if there is a fault therein, the user or the maintenance worker may disassemble each assembly and replace the damaged part, thereby saving the use costs.

In the embodiment, the circuit board assembly 20, the heating atomization assembly 30, and the battery assembly 40 are set as independent models. Further, the circuit board assembly 20 is fixed in the housing 10, the heating atomization assembly 30 is partially inserted into the first end 14 of the housing 10, the battery assembly 40 is at least partially received in the second end 15 of the housing 10, and the heating atomization assembly 30 and the battery assembly 40 are detachably connected to the circuit board assembly 20, such that the heating atomization assembly 30 and the battery assembly 40 may be taken out of the housing 10. In this way, it is possible to modularize the electronic heating non-combustion atomization device 100, such that it is convenient to assemble and disassemble each model. Thus, it is convenient to maintain the electronic heating non-combustion atomization device 100 and replace a component of the electronic heating non-combustion atomization device, thereby prolonging the service life of the electronic heating non-combustion atomization device 100.

Optionally, the heating atomization assembly 30 and/or the battery assembly 40 are detachably connected to the housing 10.

The heating atomization assembly 30 may be detachably connected to the housing 10, such that the heating atomization assembly 30 may be relatively fixed and separated from the housing 10. Similar to the above, when the heating atomization assembly 30 is connected to the housing 10, the heating atomization assembly 30 is electrically connected to the circuit board assembly 20 through the conductive terminal, the contactor, or the like. When the heating atomization assembly 30 is detachable from the housing 10, the electrical connection between the heating atomization assembly 30 and the circuit board assembly 20 is correspondingly broken down.

The battery assembly 40 may be detachably connected to the housing 10, such that the battery assembly 40 may be relatively fixed and separated from the housing 10. Similar to the above, when the battery assembly 40 is connected to the housing 10, the battery assembly 40 is electrically connected to the circuit board assembly 20 through the conductive terminal, the contactor, or the like. When the battery assembly 40 is detachable from the housing 10, the electrical connection between the battery assembly 40 and the circuit board assembly 20 is correspondingly broken down.

Referring further to FIG. 2, optionally, the outer circumference of the heating atomization assembly 30 is arranged with a first positioning bump 35. A first positioning groove 12 is defined on the first end 14 of the housing 10. The first positioning bump 35 may be engaged in the first positioning groove 12.

The first positioning bump 35 is disposed on the outer circumference of the heating atomization assembly 30, the first positioning groove 12 is defined in first end 14 of the housing 10, and the first positioning bump 35 is engaged in the first positioning groove 12, such that the first positioning bump 35 is firmly connected with the first positioning groove 12, thereby improving the user experience during the operation of an assembly.

Optionally, the outer circumference of the battery assembly 40 is arranged with a second positioning bump 44. A second positioning groove 13 is defined on the second end 15 of the housing 10. The second positioning bump 44 may be engaged in the second positioning groove 13.

In an embodiment, the first positioning bump 35 and the second positioning bump 44 may be in a curved shape, such as the semicircle. Accordingly, the first positioning groove 12 may also be in the curved shape, such as the semicircle.

In other embodiments, the first positioning bump 35 and the second positioning bump 44 may also be in other curved shape, square shape, or the like. Accordingly, the first positioning groove 12 may also be a square groove, which is not limited herein.

The first positioning bump 35 is arranged with a curved surface facing one end surface of the housing 10. During the operation of an assembly, the curved surface of the first positioning bump 35 abuts against the end surface of the housing 10. Likewise, the second positioning bump 44 is arranged with a curved surface facing the other end surface of the housing 10. During the operation of an assembly, the curved surface of the second positioning bump 44 abuts against the other end surface of the housing 10.

Optionally, the heating atomization assembly 30 is detachably connected to the circuit board assembly 20 in the manner of magnetically connecting or snapping.

In an embodiment, the end surface of the heating atomization assembly 30 which is configured to contact the circuit board assembly 20 is arranged with a metal sheet, and the end surface of the circuit board assembly 20 which is configured to contact the heating atomization assembly 30 is arranged with a magnetic member, such as a magnet, such that the heating atomization assembly 30 may be magnetically connected to the circuit board assembly 20. Alternatively, the side surface of the heating atomization assembly 30 which is configured to contact the housing 10 is arranged with the magnetic member, such as the magnet, and the end surface of the circuit board assembly 20 which is configured to contact the heating atomization assembly 30 is arranged with the metal sheet, such that the heating atomization assembly 30 may also be magnetically connected to the circuit board assembly 20. When in use, after the heating atomization assembly 30 is inserted into the housing 10 by the user, it is convenient to assemble an assembly due to the suction of the magnetic member on the metal sheet, and the heating atomization assembly 30 may be detachably connected with the circuit board assembly 20. In addition, the metal sheet may also be the magnetic member.

In another embodiment, the heating atomization assembly 30 and the circuit board assembly 20 may be snapped together in the following ways, such as a bayonet connection, or other common snapping structures (not shown).

Optionally, the battery assembly 40 is detachably connected to the circuit board assembly 20 in the manner of magnetically connecting or snapping.

In an embodiment, the end surface of the battery assembly 40 which is configured to contact the circuit board assembly 20 is arranged with a metal sheet, and the end surface of the circuit board assembly 20 which is configured to contact the battery assembly 40 is arranged with a magnetic member, such as a magnet, such that the battery assembly 40 may be magnetically connected to the circuit board assembly 20. Alternatively, the side surface of the battery assembly 40 which is configured to contact the housing 10 is arranged with the magnetic member, such as the magnet, and the end surface of the circuit board assembly 20 which is configured to contact the battery assembly 40 is arranged with the metal sheet, such that the battery assembly 40 may also be magnetically connected to the circuit board assembly 20. When in use, after the battery assembly 40 is inserted into the housing 10 by the user, it is convenient to assemble an assembly due to the suction of the magnetic member on the metal sheet, and the battery assembly 40 may be detachably connected with the circuit board assembly 20. In addition, the metal sheet may also be the magnetic member.

In another embodiment, the battery assembly 40 and the circuit board assembly 20 may be snapped together in the following ways, such as a bayonet connection, or other common snapping structures (not shown).

Optionally, the heating atomization assembly 30 may be detachably connected to the housing 10 in the manner of magnetically connecting, snapping, or closely connecting.

In another embodiment, the end surface of the heating atomization assembly 30 which is configured to contact the circuit board assembly 20 is arranged with a metal sheet, and the inner sidewall of the housing 10 is arranged with a magnetic member, such as a magnet, such that the heating atomization assembly 30 may be magnetically connected to the housing 10. Alternatively, the side surface of the heating atomization assembly 30 which is configured to contact the circuit board assembly 20 is arranged with the magnetic member, such as the magnet, and the inner sidewall of the housing 10 is arranged with the metal sheet, such that the heating atomization assembly 30 may also be magnetically connected to the housing 10. Further, the positions of the magnetic member and the metal sheet correspond to each other, such that the heating atomization assembly 30 may be relatively fixed to the housing 10 after the heating atomization assembly 30 is received in the housing 10. In addition, the metal sheet may also be the magnetic member.

In an embodiment, the heating atomization assembly 30 and the housing 10 may be snapped together in the following ways, such as a bayonet connection (not shown), or other common snapping structures.

In another embodiment, the inner side of the housing 10 or the outer circumference of the heating atomization assembly 30 is arranged with a resilient material, such that the heating atomization assembly 30 is closely connected to the housing 10. When the heating atomization assembly 30 is at least partially received in the first end 14 of the housing 10, thereby forming interference fit, thus the heating atomization assembly 30 may be closely connected to the housing 10.

Optionally, the battery assembly 40 may be detachably connected to the housing 10 in the manner of magnetically connecting, snapping, or closely connecting.

In an embodiment, the end surface of the battery assembly 40 which is configured to contact the circuit board assembly 20 is arranged with a metal sheet, and the inner sidewall of the housing 10 is arranged with a magnetic member, such as a magnet, such that the battery assembly 40 may be magnetically connected to the housing 10. Alternatively, the side surface of the battery assembly 40 which is configured to contact the circuit board assembly 20 is arranged with the magnetic member, such as the magnet, and the inner sidewall of the housing 10 is arranged with the metal sheet, such that the battery assembly 40 may also be magnetically connected to the housing 10. Further, the positions of the magnetic member and the metal sheet correspond to each other, such that the battery assembly 40 may be relatively fixed to the housing 10 after the battery assembly 40 is received in the housing 10. In addition, the metal sheet may also be the magnetic member.

In another embodiment, the battery assembly 40 and the housing 10 may be snapped together in the following ways, such as a bayonet connection (not shown), or other common snapping structures.

In another embodiment, the inner side of the housing 10 or the outer circumference of the battery assembly 40 is arranged with a resilient material, such that the battery assembly 40 is closely connected to the housing 10. When the battery assembly 40 is at least partially received in the first end 14 of the housing 10, thereby forming interference fit, thus the battery assembly 40 may be closely connected to the housing 10.

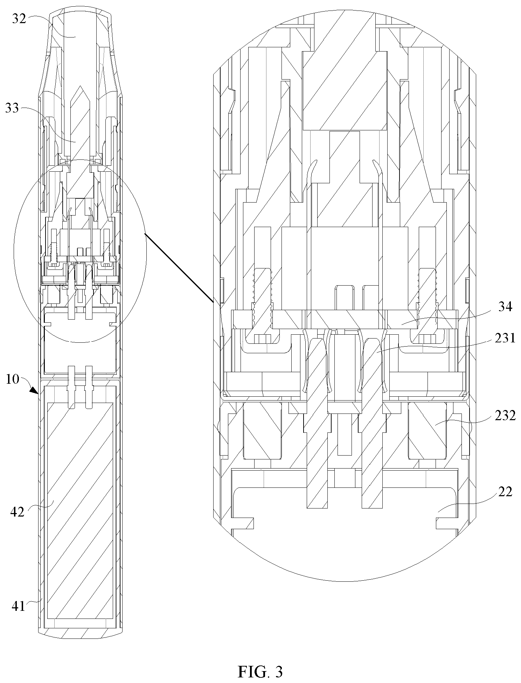

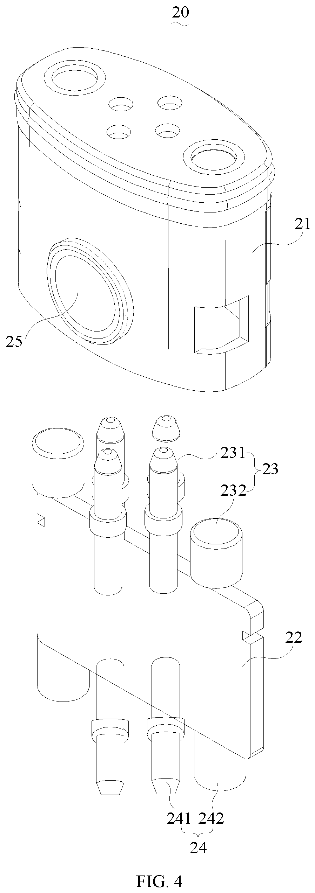

Referring to FIG. 3 and FIG. 4, FIG. 3 is a sectional schematic view of the electronic heating non-combustion atomization device according to an embodiment of the present disclosure. FIG. 4 is an exploded schematic view of a circuit board assembly of the electronic heating non-combustion atomization device according to an embodiment of the present disclosure.

Optionally, the circuit board assembly 20 includes a first support 21, a main circuit board 22, and a first connector 23.

The first support 21 is disposed in the housing 10 and is fixed to the inner sidewall of the housing 10.

The first support 21 may be integrally formed with the housing 10. The first support 21 is fixed to the inner sidewall of the housing 10 by means of gluing, or the like.

The main circuit board 22 is fixedly disposed on the first support 21.

The first connector 23 includes a first resilient electrode 231, and the first resilient electrode 231 is connected to the main circuit board 22.

The main circuit board 22 is electrically connected to the heating atomization assembly 30 via the first resilient electrode 231.

In an embodiment, the first connector 23 further includes a first magnetic member 232, the first magnetic member 232 may be fixed on the first support 21, and the end surface of the heating atomization assembly 30 is arranged with a metal sheet. Further, the first magnetic member 232 is correspondingly arranged with the metal sheet, such that the heating atomization assembly 30 and the circuit board assembly 20 may be detachably connected by the magnetic attraction between the first magnetic member 232 and the metal sheet, thus the heating atomization assembly 30 may be magnetically connected to the circuit board assembly 20.

Optionally, the circuit board assembly 20 further includes a second connector 24. The second connector 24 includes a second resilient electrode 241, and the second resilient electrode 241 is connected to the main circuit board 22.

The battery assembly 40 includes a casing 41 and a battery 42. The battery 42 is disposed in the casing 41. One of the terminals 43 of the battery 42 is electrically connected to the second resilient electrode 241.

In an embodiment, the second connector 24 further includes a second magnetic member 242, the second magnetic member 242 may be fixed on the first support 21, and the end surface of the battery assembly 40 is arranged with a metal sheet. Further, the second magnetic member 242 is correspondingly arranged with the metal sheet, such that the battery assembly 40 and the circuit board assembly 20 may be detachably connected by the magnetic attraction between the second magnetic member 242 and the metal sheet, thus the battery 42 may be magnetically connected to the circuit board assembly 20. In addition, after the second magnetic member 242 is connected to the metal sheet, the second resilient electrode 241 is correspondingly in contact with the one of the terminals 43 of the battery 42.

The circuit board assembly 20 further includes a key 25. A key hole 11 is defined on the side surface of the housing 10, and the key 25 may be exposed through the key hole 11.

The key 25 may be connected to the main circuit board 22, and the electronic heating non-combustion atomization device 100 is opening/closing, and the adjustment of mode and gear may be controlled by the user.

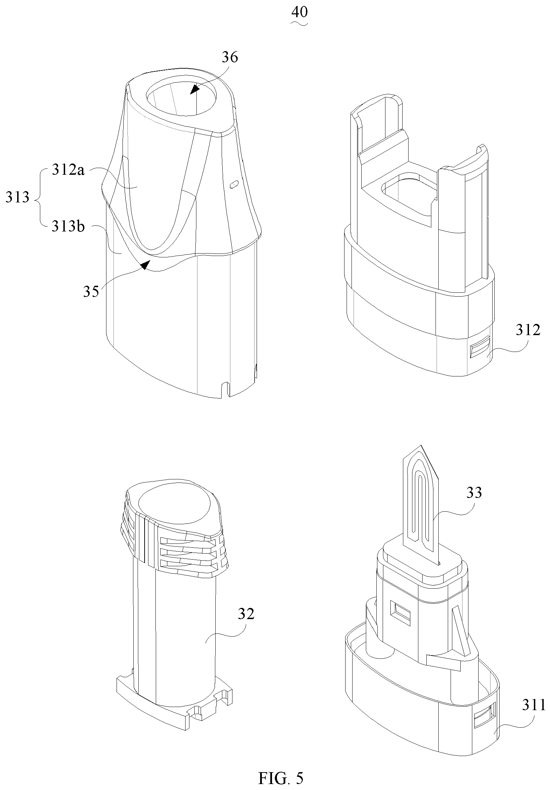

Referring to FIG. 3 and FIG. 5, FIG. 5 is an exploded structural schematic view of a heating atomization assembly of the electronic heating non-combustion atomization device.

The heating atomization assembly 30 includes a second support 31, a receiving tube 32 disposed on the second support 31, a heater 33, and a sub-circuit board 34.

The heater 33 is configured to heat the aerosol-forming substrate by extending into the receiving tube 32.

When in use, the aerosol-forming substrate, such as the smoke, is inserted into the receiving tube 32 by the user, such that the aerosol-forming substrate is heated by the heater 33, thereby generating smoke to be inhaled by the user.

The sub-circuit board 34 is electrically connected to the heater 33.

The sub-circuit board 34 is electrically connected to the main circuit board 22 via the first resilient electrode 231.

In an embodiment, after the first magnetic member 232 is connected to the metal sheet, the first resilient electrode 231 may be correspondingly in contact with the connection terminal of sub-circuit board 34.

Optionally, the second support 31 includes a heater bottom base 311, a heater support 312, and a protective cover 313.

The heater 33 is disposed on the heater bottom base 311.

Optionally, the heater 33 is detachably connected to the heater bottom base 311.

In an embodiment, the heater 33 may be engaged in the heater bottom base 311. Generally, the heater 33 belongs to the consumables. After using for a period of time, the performance of the heating plate 33 may be reduced. For example, the hardness may decline and deform, the resistance may change due to a long working time of the heating circuit, and the heating plate 33 may adhere to impurities, and so on, which may affect the smoke taste that is heated and atomized. The heater 33 is detachably connected to the heater bottom base 311, such that it is convenient to replace the heating plate 33, thereby prolonging the overall service life of the electronic heating non-combustion atomization device 100.

An end of the heater support 312 is connected to the heater bottom base 311.

Optionally, the end of the heater support 312 is detachably connected to the heater bottom base 311, and the connection part between the end of the heater support 312 and the heater bottom base 311 is arranged with a sealing member.

In another embodiment, the end of the heater support 312 may be engaged in the heater bottom base 311, and the connection part between the end of the heater support 312 and the heater bottom base 311 is sealed by a sealing ring. In this way, on the one hand, it is convenient to separate the end of the heater support 312 from the heater bottom base 311. On the other hand, it is possible to improve the sealing performance between the assembled end of the heater support 312 and the heater bottom base 311, thereby preventing the atomized liquid from flowing out from the connection part, thus the quality of the product is improved.

A through hole 36 is defined on the protective cover 313, and the receiving tube 32 is accommodated in the through hole 36 of the protective cover 313. An end of the protective cover 313 is connected to the heater support 312.

Optionally, the end of the protective cover 313 is detachably connected to the heater support 312, and the connection part between the end of the protective cover 313 and the heater support 312 is arranged with a sealing member.

In an embodiment, the end of the protective cover 313 may be engaged in t the heater support 312, and the connection part between the end and the heater support 312 is sealed by a sealing ring. In this way, on the one hand, it is convenient to separate the end of the protective cover 313 from the heater support 312. On the other hand, it is possible to improve the sealing performance between the assembled end of the protective cover 313 and the heater support 312, thereby preventing the atomized liquid from flowing out from the connection part, thus the quality of the product is improved.

The heater 33 may pass through the heater support 312 and be exposed, such that the heater 33 extends into the receiving tube 32 to heat the aerosol-forming substrate.

When in use, if it is necessary to replace the heater 33, or clean the receiving tube 32, the protective cover 313, the receiving tube 32, the heater support 312, the heater bottom base 311, and so on may be detached in sequence. The replacement method as described above is simple and easy to operate.

Optionally, the protective cover 313 includes a nozzle part 313a and a connecting part 313b. One end of the connecting part 313b is connected to the heater support 312, and the other end of the connecting part 313b is connected to the nozzle part 313a. The connecting part 313b is received in the housing 10.

The protective cover 313 includes a nozzle part 313a and a connecting part 313b. Further, the connecting part 313b is received in the housing 10, and the nozzle part 313a is exposed outside of the housing 10, such that it is convenient for the user to inhale.

In an embodiment, the electronic heating non-combustion atomization device 100 provided by the present disclosure may be applied in the atomizing inhalation therapy industry, where the atomizing inhalation therapy is the output of a drug in the form of an aerosol through different devices and into the body with breathing. Aerosol is a dispersion system consisting of solid particles or liquid particles suspended in air or air, which can be transported with air flow. In the atomizing inhalation therapy, the selection of a suitable device and the correct inhalation method have a great impact on the therapeutic effect. The atomization device provided by the present disclosure may be ally applied to devices such as quantitative pressure aerosols, dry powder inhalers, jet atomizers, etc.

In another embodiment, the electronic heating non-combustion atomization device 100 provided by the present disclosure may be applied as an electronic heating non-combustion atomization device, such as a smoking device.

In the embodiments of the present disclosure, the circuit board assembly, the heating atomization assembly, and the battery assembly are set as independent models. The battery assembly is fixed to the housing, the heating atomization assembly is at least partially received in the first end of the housing, the battery assembly is at least partially received in the second end of the housing, the heating atomization assembly and the battery assembly are detachably connected to the circuit board assembly so as to be taken out of the housing. Therefore, it is convenient to assemble and disassemble each model, and it is convenient to maintain the electronic heating non-combustion atomization device and replace the component of the electronic heating non-combustion atomization device, thereby prolonging the service life of the electronic heating non-combustion atomization device.

The above is only an implementation of the present disclosure, not to limit the scope of the present disclosure.

## Claims

1. An electronic heating non-combustion atomization device (100), comprising:
a hollow housing (10);
a heating atomization assembly (30) configured to heat an aerosol-forming substrate;
a battery assembly (40) configured to supply power; and
a circuit board assembly (20) fixedly disposed in the housing (10);
wherein the heating atomization assembly (30) is at least partially received in a first end (14) of the housing (10), and the heating atomization assembly (30) is detachably connected to the circuit board assembly (20) so as to be taken out of the housing (10); and
the battery assembly (40) is at least partially received in a second end (15) of the housing (10), and the battery assembly (40) is detachably connected to the circuit board assembly (20) so as to be taken out of the housing (10);
wherein the circuit board assembly (20) comprises:
a first support (21) disposed in the housing (10) and fixed to the inner sidewall of the housing (10);
a main circuit board (22) fixedly disposed on the first support (21); and
a first connector (23) comprising a first resilient electrode (231) connected to the main circuit board (22);
wherein the heating atomization assembly (30) comprises a second support (31), a receiving tube (32) disposed on the second support (31), and a heater (33); and
the heater (33) is configured to heat an aerosol-forming substrate by extending into the receiving tube (32);
**characterized in that** the heating atomization assembly (30) further comprises a sub-circuit board (34), wherein the sub-circuit board (34) is electrically connected to the heater (33), and the sub-circuit board (34) is electrically connected to the main circuit board (22) via the first resilient electrode (231).

2. The electronic heating non-combustion atomization device (100) according to claim 1, wherein the heating atomization assembly (30) is detachably connected to the housing (10); and/or
the battery assembly (40) is detachably connected to the housing (10).

3. The electronic heating non-combustion atomization device (100) according to claim 2, wherein the end surface of the heating atomization assembly (30), which is configured to contact the circuit board assembly (20), is arranged with one of a metal sheet and a magnetic member, and the inner sidewall of the housing (10) is arranged with the other one of the metal sheet and the magnetic member; and
the positions of the magnetic member and the metal sheet correspond to each other.

4. The electronic heating non-combustion atomization device (100) according to claim 2, wherein the end surface of the battery assembly (40), which is configured to contact the circuit board assembly (20), is arranged with one of a metal sheet and a magnetic member, and the inner sidewall of the housing (10) is arranged with the other one of the metal sheet and the magnetic member; and
the positions of the magnetic member and the metal sheet correspond to each other.

5. The electronic heating non-combustion atomization device (100) according to claim 1, wherein the outer circumference of the heating atomization assembly (30) is arranged with a first positioning bump (35), a first positioning groove (12) is defined on the first end (14) of the housing (10), and the first positioning bump (35) is engaged in the first positioning groove (12); and/or
the outer circumference of the battery assembly (40) is arranged with a second positioning bump (44), a second positioning groove (13) is defined on the second end (15) of the housing (10), and the second positioning bump (44) is engaged in the second positioning groove (13).

6. The electronic heating non-combustion atomization device (100) according to claim 1, wherein the heating atomization assembly (30) is detachably connected to the circuit board assembly (20) in the manner of magnetically connecting or snapping; and/or
the battery assembly (40) is detachably connected to the circuit board assembly (20) in the manner of magnetically connecting or snapping.

7. The electronic heating non-combustion atomization device (100) according to claim 6, wherein the end surface of the heating atomization assembly (30), which is configured to contact the circuit board assembly (20), is arranged with a metal sheet, and the end surface of the circuit board assembly (20), which is configured to contact the heating atomization assembly (30), is arranged with a magnetic member; or
the side surface of the heating atomization assembly (30) which is configured to contact the circuit board assembly (20), is arranged with the magnetic member, and the end surface of the circuit board assembly (20), which is configured to contact the heating atomization assembly (30), is arranged with the metal sheet.

8. The electronic heating non-combustion atomization device (100) according to claim 6, wherein the end surface of the battery assembly (40), which is configured to contact the circuit board assembly (20), is arranged with a metal sheet, and the end surface of the circuit board assembly (20), which is configured to contact the battery assembly (40), is arranged with a magnetic member; or
the side surface of the battery assembly (40), which is configured to contact to the circuit board assembly (20), is arranged with the magnetic member, and the end surface of the circuit board assembly (20), which is configured to contact the battery assembly (40), is arranged with the metal sheet.

9. The electronic heating non-combustion atomization device (100) according to claim 1, wherein the second support (31) comprises:
a heater bottom base (311), with the heater (33) being disposed thereon;
a heater support (312), with its end connected to the heater bottom base (311); and
a protective cover (313) defining a through hole (36), the receiving tube (32) being accommodated in the through hole (36) and the end of the protective cover (313) connected to the heater support (312);
wherein the heater (33) passes through the heater support (312) and is exposed, such that the heater (33) extends into the receiving tube (32) to heat the aerosol-forming substrate.

10. The electronic heating non-combustion atomization device (100) according to claim 9, wherein the heater (33) is detachably connected to the heater bottom base (311);
the end of the heater support (312) is detachably connected to the heater bottom base (311), and the connection part between the end of the heater support (312) and the heater bottom base (311) is arranged with a sealing member; and
the end of the protective cover (313) is detachably connected to the heater support (312), and the connection part between the end of the protective cover (313) and the heater support (312) is arranged with a sealing member.

11. The electronic heating non-combustion atomization device (100) according to claim 9, wherein the protective cover comprises a nozzle part (313a) and a connecting part (313b), one end of the connecting part (313b) is connected to the heater support (312), the other end of the connecting part (313b) is connected to the nozzle part (313a), and the connecting part (313b) is received in the housing (10).

12. The electronic heating non-combustion atomization device (100) according to claim 1, wherein the circuit board assembly (20) further comprises a second connector (24) comprising a second resilient electrode (241), and the second resilient electrode (241) is connected to the main circuit board (22); and
the battery assembly (40) comprises a casing (41) and a battery (42), the battery (42) is disposed in the casing (41), and one of the terminals (43) of the battery (42) is electrically connected to the second resilient electrode (241).

13. The electronic heating non-combustion atomization device (100) according to any one of claims 1-12, wherein the circuit board assembly (20) further comprises a key (25), a key hole (11) is defined on the side surface of the housing (10), and the key (25) is exposed through the key hole (11).

## Patentansprüche

1. Eine verbrennungsfreie Zerstäubungsvorrichtung mit elektronischer Heizung (100), umfassend:
ein hohles Gehäuse (10);
eine Heizzerstäubungsanordnung (30), die dazu ausgelegt ist, ein aerosolerzeugendes Substrat zu erhitzen;
eine Batterieanordnung (40), die dazu ausgelegt ist, elektrische Energie bereitzustellen; und
eine Leiterplattenanordnung (20), die fest in dem Gehäuse (10) angeordnet ist;
wobei
die Heizzerstäubungsanordnung (30) zumindest teilweise in einem ersten Ende (14) des Gehäuses (10) aufgenommen ist, und die Heizzerstäubungsanordnung (30) lösbar mit der Leiterplattenanordnung (20) verbunden ist, sodass sie aus dem Gehäuse (10) entnommen werden kann; und
die Batterieanordnung (40) zumindest teilweise in einem zweiten Ende (15) des Gehäuses (10) aufgenommen ist, und die Batterieanordnung (40) lösbar mit der Leiterplattenanordnung (20) verbunden ist, sodass sie aus dem Gehäuse (10) entnommen werden kann;
wobei die Leiterplattenanordnung (20) umfasst:
einen ersten Träger (21), der in dem Gehäuse (10) angeordnet und an der Innenseitenwand des Gehäuses (10) befestigt ist;
eine Hauptleiterplatte (22), die fest auf dem ersten Träger (21) angeordnet ist; und
einen ersten Verbinder (23), der eine erste elastische Elektrode (231) umfasst, die mit der Hauptleiterplatte (22) verbunden ist;
wobei die Heizzerstäubungsanordnung (30) einen zweiten Träger (31), ein auf dem zweiten Träger (31) angeordnetes Aufnahmerohr (32) und ein Heizelement (33) umfasst; und
das Heizelement (33) dazu ausgelegt ist, ein aerosolerzeugendes Substrat durch Hineinragen in das Aufnahmerohr (32) zu erhitzen;
**dadurch gekennzeichnet, dass**
die Heizzerstäubungsanordnung (30) weiter eine Nebenleiterplatte (34) umfasst,
wobei die Nebenleiterplatte (34) elektrisch mit dem Heizelement (33) verbunden ist, und die Nebenleiterplatte (34) über die erste elastische Elektrode (231) elektrisch mit der Hauptleiterplatte (22) verbunden ist.

2. Die verbrennungsfreie Zerstäubungsvorrichtung mit elektronischer Heizung (100) gemäß Anspruch 1,
wobei die Heizzerstäubungsanordnung (30) lösbar mit dem Gehäuse (10) verbunden ist; und/oder
die Batterieanordnung (40) lösbar mit dem Gehäuse (10) verbunden ist.

3. Die verbrennungsfreie Zerstäubungsvorrichtung mit elektronischer Heizung (100) gemäß Anspruch 2,
wobei auf der Endfläche der Heizzerstäubungsanordnung (30), die dazu ausgelegt ist, mit der Leiterplattenanordnung (20) in Kontakt zu treten, eines von einem Metallblech und einem Magnetelement angeordnet ist, und auf der Innenseitenwand des Gehäuses (10) das andere von dem Metallblech und dem Magnetelement angeordnet ist; und
die Positionen des Magnetelements und des Metallblechs einander entsprechen.

4. Die verbrennungsfreie Zerstäubungsvorrichtung mit elektronischer Heizung (100) gemäß Anspruch 2,
wobei auf der Endfläche der Batterieanordnung (40), die dazu ausgelegt ist, mit der Leiterplattenanordnung (20) in Kontakt zu treten, eines von einem Metallblech und einem Magnetelement angeordnet ist, und auf der Innenseitenwand des Gehäuses (10) das andere von dem Metallblech und dem Magnetelement angeordnet ist; und
die Positionen des Magnetelements und des Metallblechs einander entsprechen.

5. Die verbrennungsfreie Zerstäubungsvorrichtung mit elektronischer Heizung (100) gemäß Anspruch 1,
wobei auf dem Außenumfang der Heizzerstäubungsanordnung (30) ein erster Positionierungsvorsprung (35) angeordnet ist, auf dem ersten Ende (14) des Gehäuses (10) eine erste Positionierungsnut (12) ausgebildet ist, und der erste Positionierungsvorsprung (35) in die erste Positionierungsnut (12) eingreift; und/oder
auf dem Außenumfang der Batterieanordnung (40) ein zweiter Positionierungsvorsprung (44) angeordnet ist, auf dem zweiten Ende (15) des Gehäuses (10) eine zweite Positionierungsnut (13) ausgebildet ist, und der zweite Positionierungsvorsprung (44) in die zweite Positionierungsnut (13) eingreift.

6. Die verbrennungsfreie Zerstäubungsvorrichtung mit elektronischer Heizung (100) gemäß Anspruch 1,
wobei die Heizzerstäubungsanordnung (30) in einer magnetischen Verbindung oder durch Verrastung lösbar mit der Leiterplattenanordnung (20) verbunden ist; und/oder
die Batterieanordnung (40) in einer magnetischen Verbindung oder durch Verrastung lösbar mit der Leiterplattenanordnung (20) verbunden ist.

7. Die verbrennungsfreie Zerstäubungsvorrichtung mit elektronischer Heizung (100) gemäß Anspruch 6,
wobei auf der Endfläche der Heizzerstäubungsanordnung (30), die dazu ausgelegt ist, mit der Leiterplattenanordnung (20) in Kontakt zu treten, ein Metallblech angeordnet ist, und auf der Endfläche der Leiterplattenanordnung (20), die dazu ausgelegt ist, mit der Heizzerstäubungsanordnung (30) in Kontakt zu treten, ein Magnetelement angeordnet ist; oder
auf der Seitenfläche der Heizzerstäubungsanordnung (30), die dazu ausgelegt ist, mit der Leiterplattenanordnung (20) in Kontakt zu treten, das Magnetelement angeordnet ist, und auf der Endfläche der Leiterplattenanordnung (20), die dazu ausgelegt ist, mit der Heizzerstäubungsanordnung (30) in Kontakt zu treten, das Metallblech angeordnet ist.

8. Die verbrennungsfreie Zerstäubungsvorrichtung mit elektronischer Heizung (100) gemäß Anspruch 6,
wobei auf der Endfläche der Batterieanordnung (40), die dazu ausgelegt ist, mit der Leiterplattenanordnung (20) in Kontakt zu treten, ein Metallblech angeordnet ist, und auf der Endfläche der Leiterplattenanordnung (20), die dazu ausgelegt ist, mit der Batterieanordnung (40) in Kontakt zu treten, ein Magnetelement angeordnet ist; oder
auf der Seitenfläche der Batterieanordnung (40), die dazu ausgelegt ist, mit der Leiterplattenanordnung (20) in Kontakt zu treten, das Magnetelement angeordnet ist, und auf der Endfläche der Leiterplattenanordnung (20), die dazu ausgelegt ist, mit der Batterieanordnung (40) in Kontakt zu treten, das Metallblech angeordnet ist.

9. Die verbrennungsfreie Zerstäubungsvorrichtung mit elektronischer Heizung (100) gemäß Anspruch 1,
wobei die zweite Trägeranordnung (31) umfasst:
eine Heizbodenbasis (311), auf der das Heizelement (33) angeordnet ist;
einen Heizerträger (312), dessen Ende mit der Heizbodenbasis (311) verbunden ist; und
eine Schutzabdeckung (313), die eine Durchgangsöffnung (36) definiert, wobei das Aufnahmerohr (32) in der Durchgangsöffnung (36) aufgenommen ist und das Ende der Schutzabdeckung (313) mit dem Heizerträger (312) verbunden ist;
wobei das Heizelement (33) durch den Heizerträger (312) hindurchtritt und freigelegt ist, so dass sich das Heizelement (33) in das Aufnahmerohr (32) erstreckt, um das aerosolerzeugende Substrat zu erhitzen.

10. Die verbrennungsfreie Zerstäubungsvorrichtung mit elektronischer Heizung (100) gemäß Anspruch 9,
wobei das Heizelement (33) lösbar mit der Heizbodenbasis (311) verbunden ist; das Ende des Heizerträgers (312) lösbar mit der Heizbodenbasis (311) verbunden ist, und an der Verbindungsstelle zwischen dem Ende des Heizerträgers (312) und der Heizbodenbasis (311) ein Dichtelement angeordnet ist; und
das Ende der Schutzabdeckung (313) lösbar mit dem Heizerträger (312) verbunden ist, und an der Verbindungsstelle zwischen dem Ende der Schutzabdeckung (313) und dem Heizerträger (312) ein Dichtelement angeordnet ist.

11. Die verbrennungsfreie Zerstäubungsvorrichtung mit elektronischer Heizung (100) gemäß Anspruch 9,
wobei die Schutzabdeckung einen Düsenabschnitt (313a) und einen Verbindungsabschnitt (313b) umfasst, wobei ein Ende des Verbindungsabschnitts (313b) mit dem Heizerträger (312) verbunden ist, das andere Ende des Verbindungsabschnitts (313b) mit dem Düsenabschnitt (313a) verbunden ist, und der Verbindungsabschnitt (313b) in dem Gehäuse (10) aufgenommen ist.

12. Die verbrennungsfreie Zerstäubungsvorrichtung mit elektronischer Heizung (100) gemäß Anspruch 1,
wobei die Leiterplattenanordnung (20) ferner einen zweiten Verbinder (24) umfasst, der eine zweite elastische Elektrode (241) umfasst, und die zweite elastische Elektrode (241) mit der Hauptleiterplatte (22) verbunden ist; und
die Batterieanordnung (40) ein Batteriegehäuse (41) und eine Batterie (42) umfasst, wobei die Batterie (42) in dem Batteriegehäuse (41) angeordnet ist und einer der Anschlüsse (43) der Batterie (42) elektrisch mit der zweiten elastischen Elektrode (241) verbunden ist.

13. Die verbrennungsfreie Zerstäubungsvorrichtung mit elektronischer Heizung (100) gemäß einem der Ansprüche 1 bis 12,
wobei die Leiterplattenanordnung (20) ferner eine Taste (25) umfasst, auf der Seitenfläche des Gehäuses (10) ein Tastenloch (11) ausgebildet ist, und die Taste (25) durch das Tastenloch (11) hindurch freiliegt.

## Revendications

1. Un dispositif de nébulisation sans combustion à chauffage électronique (100), comprenant :
un boîtier creux (10) ;
un ensemble de nébulisation par chauffage (30) configuré pour chauffer un substrat formant un aérosol ;
un ensemble de batterie (40) configuré pour fournir de l'énergie ;
et un ensemble de carte de circuit imprimé (20) disposé de manière fixe dans le boîtier (10) ;
dans lequel l'ensemble de nébulisation par chauffage (30) est reçu au moins partiellement dans une première extrémité (14) du boîtier (10), et l'ensemble de nébulisation par chauffage (30) est connecté de manière amovible à l'ensemble de carte de circuit imprimé (20) de manière à pouvoir être retiré du boîtier (10) ; et
l'ensemble de batterie (40) est reçu au moins partiellement dans une seconde extrémité (15) du boîtier (10), et l'ensemble de batterie (40) est connecté de manière amovible à l'ensemble de carte de circuit imprimé (20) de manière à pouvoir être retiré du boîtier (10) ;
dans lequel l'ensemble de carte de circuit imprimé (20) comprend :
un premier support (21) disposé dans le boîtier (10) et fixé à la paroi latérale interne du boîtier (10) ;
une carte de circuit imprimé principale (22) fixée sur le premier support (21) ;
et un premier connecteur (23) comprenant une première électrode élastique (231) connectée à la carte de circuit imprimé principale (22) ;
dans lequel l'ensemble de nébulisation par chauffage (30) comprend un second support (31), un tube de réception (32) disposé sur le second support (31), et un élément chauffant (33) ;
et l'élément chauffant (33) est configuré pour chauffer un substrat formant un aérosol en s'étendant dans le tube de réception (32) ;
**caractérisé en ce que**
l'ensemble de nébulisation par chauffage (30) comprend en outre une sous-carte de circuit imprimé (34),
dans lequel la sous-carte de circuit imprimé (34) est électriquement connectée à l'élément chauffant (33), et la sous-carte de circuit imprimé (34) est électriquement connectée à la carte de circuit imprimé principale (22) via la première électrode élastique (231).

2. Le dispositif de nébulisation sans combustion à chauffage électronique (100) selon la revendication 1,
dans lequel l'ensemble de nébulisation par chauffage (30) est connecté de manière amovible au boîtier (10) ; et/ou
l'ensemble de batterie (40) est connecté de manière amovible au boîtier (10).

3. Le dispositif de nébulisation sans combustion à chauffage électronique (100) selon la revendication 2,
dans lequel est disposé, sur la surface d'extrémité de l'ensemble de nébulisation par chauffage (30) configurée pour entrer en contact avec l'ensemble de carte de circuit imprimé (20), l'un parmi une plaque métallique et un élément magnétique, tandis que l'autre parmi la plaque métallique et l'élément magnétique est disposé sur la paroi latérale interne du boîtier (10) ; et
les positions de l'élément magnétique et de la plaque métallique correspondent l'une à l'autre.

4. Le dispositif de nébulisation sans combustion à chauffage électronique (100) selon la revendication 2,
dans lequel est disposé, sur la surface d'extrémité de l'ensemble de batterie (40) configurée pour entrer en contact avec l'ensemble de carte de circuit imprimé (20), l'un parmi une plaque métallique et un élément magnétique, tandis que l'autre parmi la plaque métallique et l'élément magnétique est disposé sur la paroi latérale interne du boîtier (10) ; et
les positions de l'élément magnétique et de la plaque métallique correspondent l'une à l'autre.

5. Le dispositif de nébulisation sans combustion à chauffage électronique (100) selon la revendication 1,
dans lequel la circonférence extérieure de l'ensemble de nébulisation par chauffage (30) est pourvue d'une première saillie de positionnement (35), une première rainure de positionnement (12) étant formée sur la première extrémité (14) du boîtier (10), et la première saillie de positionnement (35) étant engagée dans la première rainure de positionnement (12) ; et/ou
la circonférence extérieure de l'ensemble de batterie (40) étant pourvue d'une seconde saillie de positionnement (44), une seconde rainure de positionnement (13) étant formée sur la seconde extrémité (15) du boîtier (10), et la seconde saillie de positionnement (44) étant engagée dans la seconde rainure de positionnement (13).

6. Le dispositif de nébulisation sans combustion à chauffage électronique (100) selon la revendication 1,
l'ensemble de nébulisation par chauffage (30) étant connecté de manière amovible à l'ensemble de carte de circuit imprimé (20) par connexion magnétique ou par encliquetage ; et/ou
l'ensemble de batterie (40) étant connecté de manière amovible à l'ensemble de carte de circuit imprimé (20) par connexion magnétique ou par encliquetage..

7. Le dispositif de nébulisation sans combustion à chauffage électronique (100) selon la revendication 6,
dans lequel sur la surface d'extrémité de l'ensemble de nébulisation par chauffage (30), configurée pour entrer en contact avec l'ensemble de carte de circuit imprimé (20), est disposée une plaque métallique, et sur la surface d'extrémité de l'ensemble de carte de circuit imprimé (20), configurée pour entrer en contact avec l'ensemble de nébulisation par chauffage (30), est disposé un élément magnétique ; ou
la surface latérale de l'ensemble de nébulisation par chauffage (30), configurée pour entrer en contact avec l'ensemble de carte de circuit imprimé (20), est pourvue de l'élément magnétique, et la surface d'extrémité de l'ensemble de carte de circuit imprimé (20), configurée pour entrer en contact avec l'ensemble de nébulisation par chauffage (30), est pourvue de la plaque métallique.

8. Le dispositif de nébulisation sans combustion à chauffage électronique (100) selon la revendication 6,
dans lequel sur la surface d'extrémité de l'ensemble de batterie (40), configurée pour entrer en contact avec l'ensemble de carte de circuit imprimé (20), est disposée une plaque métallique, et sur la surface d'extrémité de l'ensemble de carte de circuit imprimé (20), configurée pour entrer en contact avec l'ensemble de batterie (40), est disposé un élément magnétique ; ou
la surface latérale de l'ensemble de batterie (40), configurée pour entrer en contact avec l'ensemble de carte de circuit imprimé (20), est pourvue de l'élément magnétique, et la surface d'extrémité de l'ensemble de carte de circuit imprimé (20), configurée pour entrer en contact avec l'ensemble de batterie (40), est pourvue de la plaque métallique.

9. Le dispositif de nébulisation sans combustion à chauffage électronique (100) selon la revendication 1,
dans lequel le second support (31) comprend :
une base inférieure de chauffage (311), sur laquelle est disposé l'élément chauffant (33) ;
un support de chauffage (312), dont une extrémité est reliée à la base inférieure de chauffage (311) ;
et un couvercle de protection (313) définissant une ouverture traversante (36), le tube de réception (32) étant logé dans l'ouverture traversante (36) et l'extrémité du couvercle de protection (313) étant reliée au support de chauffage (312) ;
dans lequel l'élément chauffant (33) traverse le support de chauffage (312) et est exposé, de sorte que l'élément chauffant (33) s'étend dans le tube de réception (32) afin de chauffer le substrat formant un aérosol.

10. Le dispositif de nébulisation sans combustion à chauffage électronique (100) selon la revendication 9,
l'élément chauffant (33) étant connecté de manière amovible à la base inférieure de chauffage (311) ;
l'extrémité du support de chauffage (312) étant connectée de manière amovible à la base inférieure de chauffage (311), et une pièce d'étanchéité étant disposée au niveau de la partie de connexion entre l'extrémité du support de chauffage (312) et la base inférieure de chauffage (311) ; et
l'extrémité du couvercle de protection (313) étant connectée de manière amovible au support de chauffage (312), et une pièce d'étanchéité étant disposée au niveau de la partie de connexion entre l'extrémité du couvercle de protection (313) et le support de chauffage (312).

11. Le dispositif de nébulisation sans combustion à chauffage électronique (100) selon la revendication 9,
le couvercle de protection comprenant une partie de buse (313a) et une partie de connexion (313b), une extrémité de la partie de connexion (313b) étant reliée au support de chauffage (312), l'autre extrémité de la partie de connexion (313b) étant reliée à la partie de buse (313a), et la partie de connexion (313b) étant reçue dans le boîtier (10).

12. Le dispositif de nébulisation sans combustion à chauffage électronique (100) selon la revendication 1,
dans lequel l'ensemble de carte de circuit imprimé (20) comprend en outre un second connecteur (24) comprenant une seconde électrode élastique (241), la seconde électrode élastique (241) étant connectée à la carte de circuit imprimé principale (22) ; et
l'ensemble de batterie (40) comprend un boîtier de batterie (41) et une batterie (42), la batterie (42) étant disposée dans le boîtier de batterie (41), et une borne (43) de la batterie (42) étant électriquement connectée à la seconde électrode élastique (241).

13. Le dispositif de nébulisation sans combustion à chauffage électronique (100) selon l'une quelconque des revendications 1 à 12,
dans lequel l'ensemble de carte de circuit imprimé (20) comprend en outre une touche (25), un orifice de touche (11) étant défini sur la surface latérale du boîtier (10), la touche (25) étant exposée à travers l'orifice de touche (11).
